# EUROPEAN PATENT APPLICATION

(11) **EP 4 574 984 A1**
(43) Date of publication of application: **25.06.2025**
(21) Application number: 23219228.6
(22) Date of filing: 21.12.2023
(51) Int. Cl.: C12P 7/40, C12P 7/52, C12P 19/42, C12P 7/46, C12P 7/54

(54) **A METHOD OF PRODUCING ORGANIC ACIDS OR SALTS THEREOF**

(71) Applicant: Tirlán Limited, Kilkenny (IE)
(72) Inventor: O'NEILL, Simon, Kilkenny (IE); LAWLESS, Fergal, Kilkenny (IE); COLLINS, Michelle, Kilkenny (IE)
(74) Representative: Purdylucey Intellectual Property

(57) **Abstract**

A method of producing organic acids or salts thereof from a dairy side steam is provided. The method comprises an upstream processing process to remove minerals from the dairy side stream and subsequent fermentation using a bacterial strain capable of converting lactose to propionic acid, to provide a fermentation broth comprising one or more organic acids or organic acid salts.

## Description

### Field of the Invention

The present invention relates to methods of producing organic acids or salts thereof. Also contemplated is organic acids and organic acid salts produced according to the method of the invention and their use.

### Background of the Invention

The salts of organic acids (also referred to as organic acid salts) produced during bacterial fermentation are known to inhibit the growth of spoilage microorganisms, especially fungi, in food products. Therefore, the end-product of bacterial fermentation, containing a high level of organic salts, can be used as a food preservative to extend the shelf-life of food including baked goods. They are also used as animal feed preservatives.

Propionic acid is one such organic acid. It is a carboxylic acid with the chemical formula CH₃CH₂CO₂H. *Propionibacterium sp.,* are known to produce metabolites from sugar, including propionic acid, by fermentation. *Propionibacteria* are pleomorphic catalase- and gram-positive, anaerobic, aerotolerant bacteria that produce propionic acid as the main product via fermentation together with other products such as acetic acid. Traditionally *Propionibacteria* have been used as one of the starter cultures of Swiss-type cheese to produce the characteristic flavour and eyes. More recently, *Propionibacteria* are used in the production of food products. Both lactose and lactate are suitable carbon sources for propionic acid fermentation. The fermentation metabolism of *Propionibacteria* differs depending on the carbon source used. When sugar is available, the Embden-Meyerhof-Parnas (*EMP*) pathway is used as described in Antone U, et al, (Antone U et al., Propionic Acid Fermentation-Study of Substrates, Strains, and Antimicrobial Properties. Fermentation. 2023; 9(1):26)

One of the major limitations to current methods of producing propionic acid is the low yield. Large scale production by *Propionibacteria* can have severe inhibition of end products during cell growth. The formation of by-products, such as acetic acid and succinic acid, can also be a challenge. Organic acids, such as propionic acid and their salts are generally produced via petrochemical routes, which are not sustainable and increase the environmental pressure associated with the production of the organic acids and salts. Organic acid synthesis via microbial pathways during the fermentation of sugars such as lactose from dairy side streams is a more sustainable production route.

Propionic acid is a known by-product of biological fermentation for vitamin B12. EP3140416 discloses a method to produce a biotechnological product, such as vitamin B12, using cocultivation of *Propionibacterium* and a yeast cell, from sweet or sour whey.

Various preservatives are available on the market that increase the shelf life of bread and other bakery products. A disadvantage of some preservatives is that they change the organoleptic properties, such as taste, of the product.

EP3917334 discloses an anti-mold bakery additive, that comprises propionate and other natural organic acid salts, which is produced by microbial fermentation of carbohydrates, such as grain flours, using a consortium of three *Propionibacterium* strains, namely *Propionibacterium theonii* NCIM 2932, *Propionibacterium freudenreichii* NCIM 2111 and *Propionibacterium shermanii* NCIM 5137.

It is an aim of the current invention to solve one or more problems of the prior art and provide a method of producing an organic acid, such as propionic acid.

### Summary of the Invention

The Applicant has developed a process, typically a three-stage process, for producing organic acids or salts thereof from dairy side streams, such as dairy whey side streams. The method involves upstream processing of the dairy side stream to remove minerals (monovalent and/or multivalent ions) and provide a substrate, bacterial fermentation of the substrate to provide a fermentation broth comprising one or more organic acid salts and optional downstream processing of the fermentation broth to recover the organic acid salts.

Dairy side streams naturally contain a large amount of monovalent ions, such as sodium, potassium and chloride. Multivalent ions, such as calcium, phosphorus, sulphur, and magnesium are also present. The upstream processing step of the method of the invention that removes ions involves neutralisation and filtration, which may be used as alternative steps or used in combination.

Neutralisation of the dairy side stream is carried out with a suitable base, such as a metal hydroxide base. This step precipitates insoluble salts, typically calcium and phosphate salts, typically 50% or more, which can be removed by a suitable separation process, such as centrifugation. After removal the product is typically called a clarified product or substrate. This step may be used on its own or it may be followed by filtration.

A filtration membrane fractionation method, such as nanofiltration/diafiltration, removes a large proportion of monovalent ions from the dairy side stream. This step may be used on its own, or it may follow a neutralisation step in which the clarified substrate undergoes filtration. After filtration, the product is typically called a demineralised product. This may be fully or partially demineralised depending on whether or not clarification has taken place.

This upstream processing procedure generates a more suitable fermentation substrate by reducing the mineral content and increasing the lactose content. Additional minerals creates a higher osmotic pressure environment in the fermentation substrate that may inhibit bacterial cell growth, decrease organic acid productivity and lead to incomplete fermentations. Therefore, upstream pre-treatment can enhance the bioconversion of lactose to organic acids and their salts during fermentation improving the overall fermentation performance such as lactose to organic acid productivity, yield and conversion.

According to an aspect of the invention, there is provided a method to produce an organic acid or salt thereof, from a dairy side stream, the method comprising the steps of:
neutralising the dairy side stream to precipitate multivalent ions,
separating the precipitate to provide a clarified product,
and/or filtering the dairy side stream, or the clarified product, to provide a demineralised product,
incubating the product with a bacterium capable of converting lactose to propionic acid to provide a fermentation broth containing one or more organic acids or organic acid salts.

In an embodiment, incubation step further comprises adding one or more suitable bases to convert the organic acids to organic acid salts, to provide a fermentation broth containing one or more organic acid salts, i.e. one or more of propionates, acetates, succinates and lactates.

In an embodiment, the method further comprises recovering the organic acid or organic acid salts from the fermentation broth.

Typically, the one or more organic acid is one or more of propionic acid, acetic acid, succinic acid and lactic acid.

In an embodiment, the dairy side stream is neutralised by addition of a suitable base. Preferably, a basic metal hydroxide.

Preferably, the basic metal hydroxide is calcium hydroxide. Other metal hydroxides may be employed to neutralise the substrate and precipitate the salts, for example sodium hydroxide, magnesium hydroxide, and potassium hydroxide.

The metal hydroxide may be a liquid or in dry form, such as a dry powder.

In an embodiment, the dairy side stream is a dairy whey side stream comprising at least 30 g/L lactose.

In an embodiment, the dairy side stream is selected from a dairy whey side stream, milk protein concentrate, whey protein concentrate or milk permeate or a combination thereof.

Typically, the dairy whey side stream is selected from whey permeate (WP), whey permeate by-products such as delactosed permeate (DLP) and delactosed concentrate (DLC) and concentrated whey permeate (CWP).

The dairy side stream can be a powder or a liquid.

In an embodiment, the filtration step to remove monovalent ions may be by means of nanofiltration/diafiltration.

In an embodiment, the product is diluted 1:3 to 1:5 prior to filtration.

In an embodiment, the nanofiltration/diafiltration employs a membrane having a molecular weight cut-off of 100-400 Daltons.

In one embodiment, the nanofiltration/diafiltration employs a membrane having a molecular weight cut-off of about 150-300 Daltons.

In an embodiment, the clarified or demineralised product is passed through an indirect tubular ultra-heat treatment step to reduce the microbial load prior to fermentation. The treatment step may be carried out at a temperature of from about 90°C to about 150°C, or from about 110°C to about 125°C, and typically for a period of from about 1 second to 180 seconds, e.g. 60 seconds. In an embodiment, the treatment step is carried out at a temperature of about 105°C for about 30 seconds.

In an embodiment, the product is heated after neutralisation and prior to separation. For example, the product may be heated at 50°C to 80°C, e.g. 70°C, and incubated for about 10 to 60 minutes, e.g. 30 minutes after neutralisation. Typically, the product is mixed during heating to ensure homogenous heating.

Typically, the upstream processing steps, i.e. prior to fermentation, reduce the calcium levels in dairy side stream by at least 50%, 60% or 70%. In one embodiment, the upstream processing reduces the calcium levels by at least 50% to 80%. In one embodiment, when the stream is delactosed whey permeate, the upstream processing reduces the calcium levels in the whey by-product by 65 to 85%, ideally 70 to 75%. In one embodiment, when the stream is whey permeate, the upstream processing reduces the calcium levels by 50 to 70%, ideally 55 to 61%. % values provided are % dry weight unless otherwise indicated.

In one embodiment, the upstream processing reduces the phosphate levels in the dairy side stream by at least 60%, 70% or 80%. In one embodiment, the upstream processing reduces the phosphate levels by at least 60% to 95%. In one embodiment, when the stream is delactosed whey permeate, the upstream processing reduces the phosphate levels in by 75 to 95%, ideally 80 to 90%. In one embodiment, when the stream is whey permeate, the upstream processing reduces the calcium levels in the whey by-product by 60 to 80%, ideally 65 to 71%.

The % reduction values provided above for calcium and phosphate are based on a mg/kg parameter. Thus, if liquid DLP contains 1000 mg calcium per kg pre-treatment, and 300mg per kg post-treatment, this correlates with a 70% reduction in calcium.

In an embodiment, the bacterial strain used in fermentation is from the genus *Propionibacterium.* In an embodiment, the strain is from the species *Propionibacterium freudenreichii.* In an embodiment, the strain is from the species *Propionibacterium acidipropionici.* In an embodiment, the strain is *Propionibacterium freudenreichii ssp. shermanii* NCIMB 8099, *Propionibacterium freudenreichii ssp. freudenreichii* NCIMB 5959, *Propionibacterium freudenreichii ssp. shermanii* NCIMB 10585, *Propionibacterium acidipropionici* NCIMB 8070 or other *Propionibacteria* that can produce propionic acid from dairy streams containing lactose. Two or more strains may be used in combination.

In an embodiment, after the fermentation step, the method further comprises a step of removing bacterial cells from the fermentation broth. Typically, removal is by centrifugation but it will be appreciate that any suitable means may be used. This step provides a supernatant that comprises one or more organic acids or organic acid salts , depending on the incubation conditions used.

The supernatant comprising the organic acids or organic acid salts can be further treated or processed if desired.

In an embodiment, the supernatant is concentrated. Concentration may be by any suitable means such as evaporation or reverse osmosis.

In an embodiment, the supernatant is pasteurised and concentrated.

The end product is an organic acid (comprising propionic, acetic, succinic and lactic acid) or organic acid salt (comprising propionate, acetate, succinate and lactate) concentrate.

In an embodiment, the organic salt concentrate is mixed with a powder carrier.

In an embodiment, the powder carrier is maltodextrin.

In an embodiment, the organic salt concentrate is dried. This may be by processing through spray drying technology. The product is an organic salt powder.

In an embodiment, the method has a lactose to organic salt yield of at least 50%, 60%, 70%, 75% or 80%.

In an embodiment, the method has a substrate conversion (lactose consumption) of at least 70%, 80%, 95% or 99%.

The bacterial cells removed from the fermentation broth may be further concentrated, e.g. via drying, to produce a vitamin B12 product.

In an embodiment where it is desired to produce a vitamin B12 product as a by-product of the method of the invention, the incubation step further comprises addition of one or more precursors of vitamin B12, such as cobalt chloride and 5,6 dimethylbenzimidazole (DMBI).

In such an embodiment, the conditions are switched from anaerobic or microaerophilic conditions to aerobic conditions after producing the organic salts.

An organic acid product produced by the current invention is provided.

An organic acid salt product produced by the current invention is provided.

The product is typically made up of sodium or calcium propionate (25-50% TS), acetate (15-30% TS), succinate (5-10% TS) and lactate (0-10% TS). Other components such as minerals (5-15%), protein (2-8% TS) and residual sugars including glucose, galactose, and lactose (0-5% TS) are also present.

An aspect of the invention provides an organic acid salt product comprising (or consisting of) sodium or calcium propionate, acetate, succinate and lactate. Typically the product comprises from about 25-50% TS sodium or calcium propionate, e.g. 30% 35%, 40%, 45%, from about 15% to 30% TS acetate, e.g. 20% or 25%, from about 5% to 10% TS succinate, e.g. 6% or 8%, from about 0 to 10% TS lactate, e.g. 2%, 6% or 8%. The product may further comprise other components such as minerals (5-15% TS), protein (2-8% TS) and residual sugars including glucose, galactose, and lactose (0-5% TS). In an embodiment the product is a dry product such as a powder. The product may be one produced by the method of the current invention.

An aspect of the invention provides an organic acid product comprising (or consisting of) propionic acid, acetic acid, succinic acid and lactic acid. The product may be one produced by the method of the current invention. Typically the product comprises from about 25-45% TS propionic acid, e.g. 30% 35%, 40%, from about 10% to 25% TS acetic acid, e.g. 15% or 20%, from about 5% to 10% TS succinic acid, e.g. 6% or 8%, from about 0 to 10% TS lactic acid, e.g. 2%, 6% or 8%. The product may further comprise other components such as minerals (5-15% TS), protein (2-8% TS) and residual sugars including glucose, galactose, and lactose (0-5% TS).

A preservative comprising the organic acid salt product produced by the method of the current invention is provided.

A vitamin B12 product produced by the method of the current invention is provided. A calcium phosphate product produced by the method of the current invention is provided.

A nutritional food supplement comprising (or consisting of) the calcium phosphate product is also provided.

Other aspects and preferred embodiments of the invention are defined and described in the other claims set out below.

### Definitions

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

The term "dairy side stream" is dairy-based product containing lactose that is produced from the production of primary dairy products streams such as milk, milk proteins, cheese, lactose and whey. In an embodiment, it is one that contains at least 30g/L lactose. The term includes dairy whey side stream.

"Dairy whey side streams" or "whey by-product" means a liquid by-product of whey processing that contains at least 30 g/L lactose. It may also contain other organics and at least 3 g/L minerals. Generally, the whey by-product is produced when liquid whey is fractionated to remove protein leaving a permeate rich in lactose and minerals. The term includes whey permeate (WP), delactosed permeate (DLP), delactosed concentrate (DLC) and concentrated whey permeate (CWP). In the method of the current invention, these substrates are pre-treated to reduce the mineral content. When not pre-treated to reduce the mineral content, the whey by-product typically has a high mineral content. In one embodiment, the dairy whey stream contains at least 3 g/L, 6 g/L, 10 g/L, 15 g/L, 30 g/L, 60 g/L, 100 g/L, 200 g/L, 300 g/L minerals. Mineral concentrations for calcium, magnesium, sodium, potassium, sulphur, and phosphorus after diluting and digesting incoming samples with 5% HNOs and processing the test samples through an Agilent 720 inductively coupled plasma-optical emission spectroscopy (ICP-OES) instrument. The analytical wavelengths (nm) chosen for each element were as follows: Ca (318.127 and 422.673), Mg (280.270), Na (568.821 and 589.592), K (769.897), P (213.618) and S (181.972). Chloride concentrations were determined via titration using a Mk II 926 chloride analyser. In one embodiment, the dairy whey stream contains at least 40 g/L, 50 g/L, 60 g/L, 70 g/L, 80 g/L, 90 g/L, 100 g/L, 150 g/L, 200 g/L, 250 g/L, 300 g/L, lactose, and other organics. Lactose, glucose, galactose, and organic acid concentrations were determined on a high-performance liquid chromatography (HPLC, Agilent series 1200, Japan) system equipped with a refractive-index detector. The HPLC column used was Hi-Plex H 8 µm (300 mm x 7.7 mm) with 8.5 mM sulphuric acid as the mobile phase at a flow rate of 0.6mL/min whilst the column temperature was maintained at 50 °C.

The bacterium used in the method of the invention is one capable of converting lactose to propionic acid. Typically, it can utilise lactose to also produce other organic acids, such as acetic, succinic and lactic acid. In an embodiment, the bacterial strain used in fermentation is from the genus *Propionibacterium.* Examples of bacteria capable of bioconversion of lactose in substrates into organic acids, including propionic acid, are described in the literature, and that can be used in the method of the invention include but are not limited *Propionibacterium acidipropionici* (Jiang et al.; Enhanced propionic acid production from whey lactose with immobilized Propionibacterium acidipropionici and the role of trehalose synthesis in acid tolerance. Green Chem., 2015, 17, 250), *Propionibacterium freudenreichii, Propionibacterium thoenii, Propionibacterium jensenii* (Colomban et al.; Sequential production of propionic acid from whey permeate by sequential fermentation, ultrafilration and cell recycling, Biotechnology and Bioengineering, vol.42, (1993)), *Acidipropionibacterium acidipropionici, Acidipropionibacterium cyclohexanium, acidipropionibacterium jensenii and Acidipropionibacterium thoenii* (Antone et al.; Propionic acid fermentation - study of substrates, strains, and antimicrobial properties. Fermentation 2023, 9, 26). The bacterium is one with a minimum bioconversion activity of lactose to propionic acid of 10%. Method to test this activity are known in the art, for example lactose and propionic acid can be quantified via HPLC analysis of fermentation test samples.

When used herein the term "whey permeate" is taken to mean the following: Whey is the liquid remaining after milk has been curdled and strained. It is a by-product of the manufacture of cheese and casein. It can exist as sweet whey or acid whey. The whey may be obtained from bovine milk or milk from other mammals such as goats or sheep. Preferably, the milk is bovine milk. Whey permeate is produced by removing protein and other solid components from whey. It is generally produced by treating liquid whey to ultrafiltration. Whey permeate typically contains at least 40 g lactose per litre.

When used herein "concentrated whey permeate" (CWP) refers to a product derived from whey permeate evaporation. Typically, concentrated whey permeate contains 200-240 g lactose per litre.

When used herein "delactosed whey permeate" (DLP) is a by-product of processing of whey permeate to remove lactose. However, it has a higher amount of lactose post-filtration compared with whey permeate, and higher amounts of salts and minerals, especially chlorides and phosphates. Typically DLP contains at least 180 g lactose per litre.

When used herein the term "neutralising" means adding a base to the dairy side stream to bring the pH at or close to neutral, at which pH divalent calcium and phosphate ions in side stream and can be removed from the substrate. In a preferred embodiment, the base is a basic metal hydroxide which includes alkali metal hydroxides and alkaline earth metal hydroxides. Exemplary metal hydroxides include sodium hydroxide, potassium hydroxide, magnesium hydroxide and calcium hydroxide.

When used herein the term "metal hydroxide" refers to metal hydroxides that are basic such as sodium hydroxide, potassium hydroxide, magnesium hydroxide and calcium hydroxide. The term includes alkali metal hydroxides and Alkaline earth metal hydroxides. Specific hydroxides include sodium hydroxide, calcium hydroxide, barium hydroxide, magnesium hydroxide, and potassium hydroxide. Typically, the metal hydroxide is employed in a dry form such as a dry powder form or in solution. Typically, a sufficient amount of metal hydroxide is added to neutralise the product.

When used herein the term "Nanofiltration/Diafiltration" refers to a process of nanofiltration in which the feed is diluted prior to nanofiltration. In one embodiment, the feed is diluted 1:2, 1:3, 1:4, 1:5 or 1:6 times. In one embodiment, nanofiltration employs a membrane having a molecular weight cut-off of 100-400 Daltons, preferably 150-300 Daltons. In one embodiment, the nanofiltration/diafiltration step employs a GE Osmonic Membrane with a 150-300 Dalton cut-off.

Total solids (TS) is the sum of total dissolved solids (TDS) and total suspended solids (TSS) in a liquid or powder, i.e., dry solids content.

### Brief Description of the Figures

The invention will be more clearly understood from the following description of an embodiment thereof, given by way of example only, with reference to the accompanying drawings, in which:

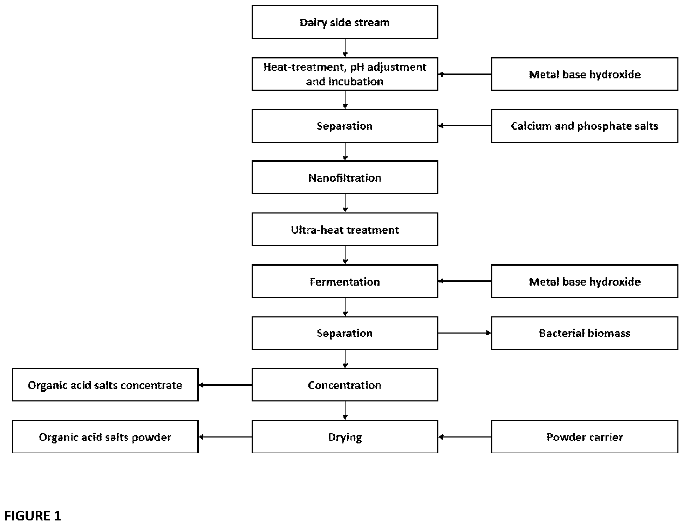

**Figure 1****:** Summary of method to produce organic acid salts from dairy side streams.

### Detailed Description of the Invention

The current invention provides a method to produce organic acids from a dairy side stream using bacterial fermentation. If desired the fermentation conditions can be such that one or more organic acid salts are produced in the fermentation broth.

Dairy side streams, such as whey used as feedstocks, for microbial fermentation in the method of the current invention, are supersaturated in the disaccharide sugar, lactose, typically containing at least 30g/L lactose. This provides a substrate for organic acid production.

The organic acids or organic acid salts produced by the method of the invention can be used as a preservative in bakery products.

As shown in Figure 1, the current method includes upstream processing step of the dairy side stream prior to fermentation. The removal of salts and ions in this step enhances bacterial fermentation.

The upstream processing can include one step of neutralisation or one step of filtration, or the two steps carried out sequentially. For example, in an embodiment, the upstream processing is a neutralisation step. In a further embodiment, the upstream processing is a filtration step. In a further embodiment, the upstream processing includes a step of neutralisation followed by a step of filtration. In a still further embodiment, the upstream processing is a filtration step followed by a neutralisation step.

The neutralisation step is carried out to precipitate the salts, such as calcium and phosphate salts, from the dairy side stream substrate.

This involves a dilution and heat-treatment step at around 60 °C and the addition of a suitable based, such as a basic metal hydroxide, such as Ca(OH)₂, NaOH, Ba(OH)₂, Mg(OH)₂ or KOH, to increase the pH of the substrate ideally to neutralise the dairy side stream at or close to around pH 7 to promote the precipitation of the mineral salts e.g. calcium and phosphate salts. It will be appreciated that any suitable base capable of providing a pH suitable to neutralise the substrate can be used. Such a base is known in the art.

Elevated temperatures, such as between 50°C to 80°C lowers the solubility of calcium and phosphate salts. Typically, this step is carried out at around 60°C. A pH range of 6 to 10 can also be targeted by adding a sufficient amount of base depending on the desired final and phosphate removal requirement.

Typically, the base is employed in a dry form or in solution. Ca(OH)₂ addition has a higher phosphate removal potential than NaOH addition as more calcium ions introduced into the dairy side stream are readily available for insoluble calcium phosphate formation. For NaOH addition, theoretically less calcium and phosphate salts are removed as the Na⁺ ions introduced from NaOH have the potential to form e.g., sodium phosphate or sodium chloride which generally remain in the soluble phase of the dairy side stream. The metal base hydroxides listed herein provide the chemical precipitation of calcium and phosphate salts, such as calcium phosphate and magnesium phosphate.

Any suitable incubation period is applied to the pH-adjusted dairy side stream to support the nucleation, crystallisation and precipitation of calcium and phosphate salts. Typically, this incubation period is about 20 minutes, or any suitable time to have the intended result.

The precipitated calcium and phosphate salts are removed by a separation process, such as centrifugation and membrane fractionation. The removal of insoluble calcium and phosphate salts by means of chemical precipitation followed by separation reduces the demineralisation that is required during the downstream process (post-fermentation). The solid by-product mainly consisting of calcium, phosphate and lactose is a suitable source of calcium and phosphate minerals for nutritional, functional, chemical, and biological applications, such as a nutritional food supplement. Once separated from the solid by product, the product is typically called a clarified product. Thus the combination of a neutralisation step and a separation step is generally referred to as a clarification process.

Following removal of the precipitated calcium and phosphate salts, the clarified product, or "supernatant" is subjected to nanofiltration/diafiltration to remove monovalent ions, if a second step is included in the upstream processing. The step facilitates the subsequent microbial fermentation and downstream processing of the fermentation broth. After ion removal, the product is typically called a demineralised product.

In an embodiment of filtration, the substrate is subjected to a volume concentration factor VCF of from 2-6 volumes, typically 3-5 volumes, or 4-5 volumes, of diafiltration water at from about 20 to 35 bar, e.g. 25 to 30 bar, transmembrane pressure. In an embodiment, the transmembrane pressure is 30 bar. In an embodiment, the temperature range for this step is from about 8°C to about 12°C, e.g. 10°C. In an embodiment, the temperature is from about 45°C to about 50°C, e.g. 48°C. A D-Series Dairy GE DK8038 C30 membrane with a molecular weight cut-off point of 150-300 Dalton for uncharged organic molecules is used for this demineralisation step.

Prior to fermentation, the substrate, which is a demineralised product, may undergo ultra-heat treatment. This is an optional step.

Bacteria (*propionibacteria* or *acidipropionibacteria*) provide a suitable inoculum for the production fermentation containing dairy side streams. In the production fermentation, the bacteria allows the bioconversion of lactose contained in the dairy side streams to organic acids, such as propionic acid.

Means of fermentation are known in the art and the methods described herein are exemplary only. It will be appreciated that the specific parameters and conditions may be optimised or changed from the exact conditions discussed in the Examples. Generally, the bacterial fermentation in the current invention is performed by a batch mode operation but can also be fed batch or a continuous fermentation operation. Methods of fermentation are known in the art. The fermentation conditions are anaerobic or microaerophilic fermentation conditions to produce the organic acids and their salts.

During bacterial fermentation organic acids are produced. The organic acids produced during bacterial fermentation can be converted into their salt form by reacting the organic acids with appropriate bases during bacterial fermentation. The appropriate bases include but are not limited to NaOH, Ca(OH)₂, Mg(OH)₂ or KOH. This is illustrated in the steps of Figure 1. The preferred organic salts are calcium and sodium organic salts produced by reacting the organic acids during bacterial fermentation with NaOH or Ca(OH)₂. Sodium and calcium propionate are the main organic salts produced during fermentation after the addition of NaOH or Ca(OH)₂ to the fermentation broth to generate these organic salts from propionic acid. Other organic salts are also produced such as calcium or sodium acetate, succinate and lactate salts after the addition of NaOH or Ca(OH)₂ to the fermentation broth to generate these organic salts from acetic, succinic and lactic acid. A combination of calcium, sodium, potassium and/or magnesium organic salts can also be produced from organic acids using more than one of the metal hydroxide bases.

After completion of the fermentation step, the fermentation broth contains mostly organic acids or organic acid salts, and bacterial cells. To remove the bacterial cells, the fermentation broth passes through a separation step, such as a clarification step, to yield a supernatant that is rich in organic acids or salts thereof. This can be called a "clarified broth".

*Propionibacterium sp.* are known producers of vitamin B12, and therefore, the removed bacterial cells can be further processed, e.g. via spray drying technology, to concentrate the vitamin B12 produced into a finished powder containing the vitamin B12. It will be appreciated that methods of concentrating bacterial biomass are known in the art. Thus, the invention also provides a method to prepare vitamin B12. In an embodiment of the method of the invention where it is desired to produce a vitamin B12 product as a by-product of the method of the invention, the incubation step may further comprise addition of one or more precursors of vitamin B12, such as cobalt chloride and 5,6 dimethylbenzimidazole (DMBI). Cobalt chloride and DMBI function as precursors for microbial vitamin B12 biosynthesis in the fermentation broth. An aerobic fermentation step is also introduced after the anaerobic or microaerophilic fermentation step. Means to introduce such a step during fermentation are known in the art. The aerobic fermentation step allows for DMBI synthesis and the formation of vitamin B12 due to the aerobic biosynthetic pathway of the microorganism. The cobalt chloride is generally added during the anaerobic fermentation step during the production of organic acids such as propionic acid as described in the method of the invention. This increases the cobalt ion concentration for cobalt chelation during vitamin B12 biosynthesis. Cobalt is required as it forms the central ion of the corrinic ring in all cobalamins (vitamin B12 family of compounds). The DMBI is generally added during the aerobic fermentation step to increase the rate of vitamin B12 biosynthesis. DMBI forms the lower ligand of cobalamin isoforms. The bacterial cells containing vitamin B12 can then be removed by centrifugation and further concentrated to produce a vitamin B12 product.

The supernatant or "clarified broth" may be further processed to provide produce a concentrated liquid comprising of organic salts or organic acids. The clarified broth is generally a diluted liquid, generally 8% TS, containing the organic salts. A concentration step is needed in the form of evaporation to provide a concentrated broth as a feed at the appropriate dry matter content for the drying process. This may be by falling film evaporation. As an alternative to evaporation, the supernatant can be passed through reverse osmosis. It will be appreciated that methods of concentrating supernatants are known in the art.

The retentate stream from reverse osmosis contains the concentrated organic salt solution.

After evaporation, the concentrated organic salt liquid can be mixed with a powder carrier, such as maltodextrin spray dried, e.g., with an inlet temperature set at 180 °C and an outlet temperature set at 90 °C. A powder containing the organic salts with a moisture content of around 3% mass is achieved after spray drying. The ideal moisture content range is ≤4.0% mass.

As described herein the product of the method of the invention is an organic acid salt product or an organic acid product. This product comprises a number of organic acid salts or organic acids. In an embodiment, the method of the invention comprises a step of recovering one organic acid from this product, such that the recovered product contains from about 75% to 100% recovered organic acid. In an embodiment, it contains about 85% or more recovered organic acid. It may have a purity of 95% or greater, 96% or greater, 97% or greater, 98% or greater, 99% or 100%. The organic acid may be any organic acid in the product of the method of the invention. For example, if a pure propionic acid product is desired that has a purity of from 95% to 100%, the method of the invention further comprises a step of recovering propionic acid from the organic acid salt or organic acid product. Methods of recovery are well known in the art.

The invention also provides a method of making a bakery product, comprising the method of the invention.

The invention further provides an organic acid salt product or concentrate produced by the method of the invention.

The invention provide use of the organic acid salt product disclosed herein or produced by the method of the invention as a preservative in bakery goods.

The invention provide use of the organic acid product disclosed herein or produced by the method of the invention as a preservative in bakery goods.

A method to prepare a bakery good preservative is provided comprising the method of the invention is provided.

A bakery good comprising the organic acid salt product, or the organic acid product described herein or produced by the method of the invention is provided.

A method to prepare vitamin B12 comprising the method of the invention is provided.

In an alternative embodiment of the method of the invention, the upstream processing steps are omitted. In other words, there is no neutralising or clarification step and no filtering step. This method comprises incubating a dairy side stream with a bacterium capable of converting lactose to propionic acid to provide a fermentation broth containing one or more organic acids or organic acid salts. All embodiments and preferred features described herein regarding fermentation and subsequent steps apply equally to this method.

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLES

### Upstream processing steps

The dairy whey side stream substrate is diluted to 12% total solids (TS) (w/w). If the dairy whey side stream is ≤12% TS, no dilution is required. The substrate is then subjected to heat-treatment at 70 °C via a heat-exchanger. A basic metal hydroxide in the form of calcium or sodium hydroxide is added to the whey permeate to adjust to pH 7. After pH adjustment, the whey permeate is incubated for 30 minutes to assist with the precipitation of calcium and phosphate salts.

After incubation, the substrate is processed through clarification for the removal of the insoluble calcium and phosphate salts. Following clarification, the supernatant is retained for analysis and further processing.

**Table 1: Average composition of whey permeate before and after sodium and calcium hydroxide pre-treatment.**

| | **Whey permeate** | **After Ca(OH)₂ treatment** | **After NaOH treatment** |
|---|---|---|---|
| **pH** | 6.11 | 7.12 | 6.82 |
| **Calcium (% TS)** | 0.84 | 0.45 | 0.25 |
| **Magnesium (% TS)** | 0.16 | 0.09 | 0.16 |
| **Phosphorus (% TS)** | 0.77 | 0.39 | 0.41 |
| **Sulphur (% TS)** | 0.12 | 0.13 | 0.15 |
| **Potassium (% TS)** | 2.69 | 2.22 | 2.57 |
| **Sodium (% TS)** | 2.04 | 1.72 | 2.66 |
| **Chloride (% TS)** | 3.52 | 3.58 | 3.2 |
| **Lactose (% TS)** | 81.52 | 82.29 | 79.94 |

To remove monovalent ions, the clarified substrate was processed via nanofiltration/diafiltration. Feed streams are subjected to a VCF of 4 at a temperature of 10 °C or 50 °C and 30 bar transmembrane pressure. Chloride, sodium, and potassium monovalent ions have the highest % rejection during nanofiltration.

**Table 2: Average rejection of minerals and lactose after nanofiltration of clarified whey permeate.**

| | **% Rejection** |
|---|---|
| **Calcium** | 90 |
| **Magnesium** | 97 |
| **Phosphorus** | 96 |
| **Sulphur** | 98 |
| **Potassium** | 32 |
| **Sodium** | 38 |
| **Chloride** | 21 |
| **Lactose** | 96 |

The demineralised substrate was passed through an indirect tubular ultra-heat treatment step at 105 °C for 30s to reduce the microbial load of the dairy whey side stream prior to fermentation.

### Fermentation

Bacteria (*propionibacteria* or *acidipropionibacteria*) were grown on suitable culture media to allow for cell growth and multiplication to provide a suitable inoculum for the production fermentation of dairy side streams. Dairy side streams are supersaturated in the disaccharide sugar, lactose. In the production fermentation, the metabolites produced by the bacteria allows for the bioconversion of lactose contained in the dairy side streams to organic acids. Generally, the bacterial fermentation is performed by a batch mode operation but can also be fed batch or a continuous fermentation operation. The lactose concentration of the dairy whey side streams are variable due to the nature of its origin and type of dairy whey side stream used. To confirm the lactose content of the solution, each dairy whey side stream is assayed each time to determine the volumes required at each fermentation stage.

Bacterial fermentation allows for the bioconversion of lactose to organic acids. The organic acids produced during bacterial fermentation can be converted into their salt form by reacting the organic acids with appropriate bases during bacterial fermentation such as NaOH, Ca(OH)₂, Mg(OH)₂ or KOH. The preferred organic salts are calcium and sodium organic salts produced by reacting the organic acids during bacterial fermentation with NaOH or Ca(OH)₂. A combination of calcium, sodium, potassium and/or magnesium organic salts can also be produced from organic acids using more than one of the metal hydroxide bases. After completion of the fermentation, a fermentation broth is produced that contains the bio-based organic salts for downstream processing.

A biotechnological process of the invention to grow the culture and to produce organic salts from dairy whey side streams may include the following steps:
1) Primary inoculum stage to prepare culture medium.
2) Secondary inoculum stage for seed fermentation inoculum.
3) Seed fermentation stage for production fermentation inoculum.
4) Production fermentation to produce a fermentation broth containing organic salts.
5) Optional continuation of subsequent production fermentations

### 1) Primary inoculum stage

The primary inoculum stage consists of a 500 mL shake-flask pre-culture grown on liquid media, inoculated from a thawed glycerol stock containing a propionibacterium (*Propionibacterium sp.*) or acidipropionibacterium (*Acidipropionibacterium sp.*) bacterial culture.

**Table 3: Recipe for primary inoculum medium.**

| **Component** | **Amount** |
|---|---|
| DL - Sodium lactate | 20 (mL/L water) |
| Yeast extract | 10 (g/L water) |
| Casein peptone | 5 (g/L water) |
| Water | For dilution |

Add 300mL water in a beaker. Add all media components and continuously mix until all media components are solubilized. Adjust media to pH 6.5 using 1M NaOH or 1M HCl. Sterilise the media using a 0.2µm filter and transfer to a sterile Erlenmeyer flask.

Place the Erlenmeyer flask in a shake-incubator and cultivate under the operating conditions as shown in Table 4.

When the transfer criterion is reached (duration), remove the flask from the incubator and immediately transfer the broth aseptically into the fermentation vessels that contains the secondary inoculum substrate at the suitable operating conditions.

**Table 4: Operating conditions in primary inoculum stage in orbital shaker.**

| **Parameter** | **Value/Set point** |
|---|---|
| Temperature | 30°C (+/- 1 °C) |
| Shaking | 80 rpm |
| Duration | 96 h (+/- 8 h) |

### 2) Secondary inoculum stage

The secondary inoculum stage consists of a 3L non-aerated fermentation in a bioreactor. The medium composition for this stage is described in Table 5.

Filter sterilise the media components in water using a 0.2µm filter. The dairy whey side stream can also be aseptically transferred directly to the bioreactor if the solution has passed through an ultra-heat treatment step, thus, avoiding the need to filter sterilise dairy whey side stream.

Add all media aseptically to the pre-sterilised bioreactor and start agitation. Bring the medium to operating temperature. Initiate pH control using 15% (w/v) calcium hydroxide or 20% (w/w) sodium hydroxide at the desired pH set point. Aseptically inoculate the bioreactor using the primary inoculum broth (inoculum ratio ≈ 10%).

Carry out the fermentation at a temperature of 30°C (+/-1 °C) and pH 6.5 (+/- 0.1). Secondary inoculum completion occurs once there is exponential base demand/cell growth or there is >50% lactose is consumed.

**Table 5: Recipe for secondary inoculum substrate.**

| **Material** | **Concentration (g material/kg fermentation substrate)** | **Comment** |
|---|---|---|
| Yeast extract | 10 | |
| Casein peptone | 5 | |
| Ammonium sulphate | 1 | |
| Dairy whey side stream | 300 - 900 | Concentration based on initial lactose content of dairy whey side stream and final organic salt titre target |
| Water | 84 - 684 | Concentration based on initial lactose content of dairy whey side stream and final organic salt titre target |

### 3) Seed fermentation

The seed fermentation stage consists of a 20 L non-aerated fermentation in a bioreactor. The medium composition for seed fermentation is described in Table 6.

Filter sterilise the media components in water using a 0.2µm filter. The dairy whey side stream can also be aseptically transferred directly to the bioreactor if the solution has passed through an ultra-heat treatment step, thus, avoiding the need to filter sterilise dairy whey side stream.

Add all media aseptically to the pre-sterilised bioreactor and start agitation. Bring the medium to operating temperature. Initiate pH control using 15% (w/v) calcium hydroxide or 20% (w/w) sodium hydroxide at the desired pH set point. Aseptically inoculate the bioreactor using the bioreactor seed fermentation broth (inoculum ratio ≈ 10%).

Carry out the production fermentation at a temperature of 30°C (+/- 1 °C) and pH 6.5 (+/- 0.1).. The seed fermentation can be used as an inoculum (inoculum ratio ≈ 10%) for the production fermentation in a 200 L bioreactor using the same methodology once there is exponential base demand/cell growth or there is >50% lactose is consumed.

**Table 6: Recipe for seed and production fermentation substrate.**

| **Material** | **Concentration (g material/kg fermentation substrate)** | **Comment** |
|---|---|---|
| Yeast extract | 5 | |
| Casein peptone | 2.5 | |
| Ammonium sulphate | 1 | |
| Dairy whey side stream | 300 - 900 | Concentration based on initial lactose content of dairy whey side stream and final organic salt titre target |
| Water | 84 - 684 | Concentration based on initial lactose content of dairy whey side stream and final organic salt titre target |

### 4) Production fermentation

The seed fermentation stage consists of a 20 L non-aerated fermentation in a bioreactor.

Heat sterilise the media and sterilise the bioreactor in place at 121 °C for 15 mins. The dairy whey side stream can also be aseptically transferred directly to the bioreactor if the solution has passed through an ultra-heat treatment step, thus, avoiding the need to filter sterilise dairy whey side stream. The target starting lactose concentration for the production fermentation substrate is 60 g/L.

Add all media aseptically to the pre-sterilised bioreactor and start agitation. Bring the medium to operating temperature. Initiate pH control using 15% (w/v) calcium hydroxide or 20% (w/w) sodium hydroxide at the desired pH set point. Aseptically inoculate the bioreactor using the bioreactor seed fermentation broth (inoculum ratio ≈ 10%).

Carry out the production fermentation at a temperature of 30°C (+/- 1 °C) and pH 6.5 (+/- 0.5).

Upon fermentation completion, base addition can be halted once approx. 95% lactose is consumed.

After completion of the 200 L production fermentation, the fermentation is harvested when the lactose is consumed, and base demand is no longer required. Otherwise, repeated batch fermentations can continue and scale-up can progress as per seed fermentation transfer criteria.

### RESULTS

### Fermentation performance

Typical fermentation performance is shown in Table 7.

**Table 7: Minimum, average, and maximum values for organic salts titre (g/L), organic salts productivity (g/L/h), substrate conversion (%), lactose to organic salts yield (%) and propionate, acetate, succinate, and lactate organic salt content of fermentation broth (% TS).**

| **Variable** | **Unit** | **Maximum** | **Average** | **Minimum** |
|---|---|---|---|---|
| Organic acid salts titre | g/L | 62.5 | 55.6 | 48.0 |
| Organic acid salts productivity | g/L/h | 0.6 | 0.4 | 0.3 |
| Substrate conversion (lactose consumption) | % | 100 | 97.5 | 94.7 |
| Lactose to organic acid salts yield | % | 86.9 | 75.8 | 63.8 |
| Propionate* | % TS | 47.2 | 40.7 | 28.7 |
| Acetate* | % TS | 25.3 | 19.6 | 16.4 |
| Succinate* | % TS | 7.7 | 6.4 | 5.2 |
| Lactate * | % TS | 12.1 | 2.7 | 0 |

| | | | | |
|---|---|---|---|---|
| * Calcium or sodium organic acid salts. | | | | |

### Downstream processing description

Downstream processing of the fermentation removes the bacterial cells from the fermentation broth and concentrates the supernatant to further concentrate the clarified broth. After downstream processing, a final organic salt concentrate or powder is produced that can be used as a food preservative or anti-fungal agent to extend the shelf-life of foods such as baked goods.

### Downstream processing steps

Following the completion of the production fermentation to effectively produce the organic salts at a concentration as shown in Table 7, the fermentation broth is sent for downstream processing. The fermentation broth contains mostly organic salts and bacterial cells. To remove the bacterial cells, the fermentation broth passes through a clarification step to yield a supernatant that is rich in organic salts. *Propionibacterium sp.* are known producers of vitamin B12, therefore, the bacterial cells can be further processed to dry the vitamin B12 produced and produce a finished powder containing vitamin B12.

To concentrate the clarified fermentation broth, the solution is processed through a falling film evaporator. The clarified fermentation broth is pasteurised at 72 °C for 15 seconds and cooled to 65 °C before evaporation. An anti-foam agent is added at 0.05% (v/v) before the feed solution is concentrated from 6-8% TS to 25% TS. Anti-foam is added to prevent foam formation during evaporation that can disrupt the operation of the unit operation and lead to unnecessary product losses. As an alternative, the clarified fermentation broth can be passed through reverse osmosis at a temperature of 20 °C, transmembrane pressure of 80 bar a volume concentration factor of 3-5 to concentrate the solution from 5-8% TS to 25% TS. The mentioned conditions for reverse osmosis is an example for optimal organic salt rejection and membrane flux. The retentate stream contains the concentrated organic salt solution. The concentrated fermentation broth can be further concentrated from 25% TS to 60% TS by evaporation.

After evaporation, the concentrated organic salt liquid can be mixed with a powder carrier such as maltodextrin and sent for spray drying with an inlet temperature set at 180 °C and an outlet temperature set at 90 °C. Generally, a multi-stage dryer with a belt drying, fluid-bed drying and sieving step is preferred. A powder containing the organic salts with a moisture content of 3% is achieved after the drying process.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method to produce organic acids, or salts thereof, from a dairy side stream, comprising the steps of:
neutralising the dairy side stream to precipitate multivalent ions comprising calcium and phosphate,
separating the precipitate to provide a clarified product,
and/or filtering the dairy side stream or clarified product to remove monovalent ions to provide a demineralised product,
incubating the demineralised product with a bacterial strain capable of converting lactose to propionic acid, to provide a fermentation broth comprising one or more organic acids selected from propionic acid, acetic acid and succinic acid, or salts thereof.

2. The method of Claim 1, wherein the incubation step further comprises adding one or more bases to convert the organic acids to organic acid salts to provide a fermentation broth containing one or more organic acid salts.

3. The method of Claim 1 or 2, further comprising recovering the organic acids or salts thereof from the fermentation broth.

4. The method of Claim 3, wherein the step of recovery comprises removal of the bacterial strains and concentrating the fermentation broth to produce a concentrated organic acid salt.

5. The method of Claim 2 to 4, wherein the concentrated organic acid salt is dried to form a powder.

6. The method of any one of the preceding claims wherein the side stream is neutralised by addition of a basic metal hydroxide.

7. The method of Claim 6, wherein the hydroxide is selected from calcium hydroxide, sodium hydroxide, magnesium hydroxide, and potassium hydroxide.

8. The method of any one of the preceding claims, wherein the dairy side stream comprises at least 30 g/L lactose.

9. The method of any one of the preceding claims, wherein the dairy side steam is a dairy whey side stream selected from whey permeate (WP), whey permeate by-products such as delactosed permeate (DLP and delactosed concentrate (DLC) and concentrated whey permeate (CWP).

10. The method of any one of the preceding claims, wherein the bacterial strain is from the genus *Propionibacterium.*

11. The method of Claim 10, wherein the strain is *Propionibacterium freudenreichii* or *Propionibacterium acidipropionici.*

12. The method of any one of the preceding claims wherein the incubation comprises addition of one or more suitable vitamin B12 precursors for vitamin B12 synthesis by the bacterial cells.

13. A method to produce vitamin B12 from a dairy side stream, comprising the steps of:
neutralising the dairy side stream to precipitate multivalent ions comprising calcium and phosphate,
separating the precipitate to provide a clarified product,
and/or filtering the dairy side stream or clarified product to remove monovalent ions to provide a demineralised product,
incubating the demineralised product with a bacterial strain capable of converting lactose to propionic acid in the presence of one or more vitamin B12 precursors, to provide a fermentation broth comprising one or more organic acids selected from propionic acid, acetic acid and succinic acid, or salts thereof,
recovery of the bacterial cells comprising vitamin B12 from the fermentation broth and optional recovery of vitamin B12 from the bacterial cells.

14. A method to produce organic acids or salts thereof, from a dairy side stream, comprising incubating the dairy side stream with a bacterial strain capable of converting lactose to propionic acid, to provide a fermentation broth comprising one or more organic acids selected from propionic acid, acetic acid and succinic acid, or salts thereof.
